# EUROPEAN PATENT SPECIFICATION

(11) **EP 3 046 546 B1**
(45) Date of publication and mention of the grant of the patent: **02.01.2019**
(21) Application number: 14772421.5
(22) Date of filing: 16.09.2014
(51) Int. Cl.: A61K 9/16, B01D 9/00, C30B 11/02, C30B 29/54

(54) **SYNTHESIS AND PARTICLE ENGINEERING OF COCRYSTALS**
SYNTHESE UND PARTIKELMANIPULATION VON CO-KRISTALLEN
SYNTHÈSE ET INGÉNIERIE DE PARTICULES DE COCRISTAUX

(30) Priority: 16.09.2013 PT 10716613
(43) Date of publication of application: 27.07.2016
(73) Proprietor: Hovione Holding Limited, Wanchai (HK)
(72) Inventor: DUARTE, Iris, Lisboa (PT); PEREIRA, Maria José, Lisboa (PT); PADRELA, Luis, Amadora (PT); TEMTEM, Márcio, Quinta do Conde (PT)
(74) Representative: Turner, Craig Robert
(86) International application number: PCT/GB2014/052799
(87) International publication number: WO 2015/036799

(56) References cited:
- WO-A1-01/24643
- WO-A1-2010/013035
- KR-A- 20100 103 174
- US-A1- 2007 031 580
- ILIC I ET AL: "Microparticle size control and glimepiride microencapsulation using spray congealing technology", INTERNATIONAL JOURNAL OF PHARMACEUTICS, ELSEVIER BV, NL, vol. 381, no. 2, 3 November 2009 (2009-11-03), pages 176-183, XP026643523, ISSN: 0378-5173, DOI: 10.1016/J.IJPHARM.2009.05.011 [retrieved on 2009-05-14] cited in the application
- PASSERINI N ET AL: "Controlled release of verapamil hydrochloride from waxy microparticles prepared by spray congealing", JOURNAL OF CONTROLLED RELEASE, ELSEVIER, AMSTERDAM, NL, vol. 88, no. 2, 7 March 2003 (2003-03-07), pages 263-275, XP004412759, ISSN: 0168-3659, DOI: 10.1016/S0168-3659(03)00009-9
- XU LIU ET AL: "Improving the Chemical Stability of Amorphous Solid Dispersion with Cocrystal Technique by Hot Melt Extrusion", PHARMACEUTICAL RESEARCH, KLUWER ACADEMIC PUBLISHERS-PLENUM PUBLISHERS, NL, vol. 29, no. 3, 19 October 2011 (2011-10-19), pages 806-817, XP035016527, ISSN: 1573-904X, DOI: 10.1007/S11095-011-0605-4 cited in the application
- ALHALAWEH A ET AL: "Formation of cocrystals from stoichiometric solutions of incongruently saturating systems by spray drying", CRYSTAL GROWTH AND DESIGN 20100804 AMERICAN CHEMICAL SOCIETY USA, vol. 10, no. 8, 4 August 2010 (2010-08-04) , pages 3302-3305, XP009181241, DOI: 10.1021/CG100451Q
- AMJAD ALHALAWEH ET AL: "Theophylline Cocrystals Prepared by Spray Drying: Physicochemical Properties and Aerosolization Performance", AAPS PHARMSCITECH, vol. 14, no. 1, 8 January 2013 (2013-01-08), pages 265-276, XP055152173, DOI: 10.1208/s12249-012-9883-3 cited in the application
- KEEN JUSTIN M ET AL: "Enhancing bioavailability through thermal processing", INTERNATIONAL JOURNAL OF PHARMACEUTICS, ELSEVIER BV, NL, vol. 450, no. 1, 22 April 2013 (2013-04-22), pages 185-196, XP028554182, ISSN: 0378-5173, DOI: 10.1016/J.IJPHARM.2013.04.042
- NING QIAO ET AL: "Pharmaceutical cocrystals: An overview", INTERNATIONAL JOURNAL OF PHARMACEUTICS, ELSEVIER BV, NL, vol. 419, no. 1, 23 July 2011 (2011-07-23) , pages 1-11, XP028306606, ISSN: 0378-5173, DOI: 10.1016/J.IJPHARM.2011.07.037 [retrieved on 2011-08-02]
- Cheri A. Barta ET AL: "Effects of Monoglycerides on P-Glycoprotein: Modulation of the Activity and Expression in Caco-2 Cell Monolayers", Molecular Pharmaceutics, vol. 5, no. 5, 6 October 2008 (2008-10-06) , pages 863-875, XP055361691, US ISSN: 1543-8384, DOI: 10.1021/mp800050q

## Description

This invention relates to the production of cocrystals, and particularly, but not exclusively, to a solvent-free, easy scalable, method which is compatible with continuous pharmaceutical processes, and that allows cocrystal synthesis and particle engineering in a single stage operation without the need for any downstream processing.

Since the advent of combinatorial chemistry and high throughput drug screening platforms in the 1990's, the number of new chemical entities (NCEs) reaching the pharmaceutical pipelines has increased exponentially. A major concern is that 70-90% of those molecules presenting therapeutic potential also present solubility constraints (*i.e*. BCS class II and class IV). Poor solubility determines limited bioavailability, conditioning the drug therapeutic effectiveness. To overcome this issue, and promote the successful translation of novel bioactive chemical entities towards the clinic, different engineering and formulation enabling platforms have been used such as particle size reduction, self-emulsifying drug delivery systems, cyclodextrin complexes, salt forms, cocrystals or amorphous solid dispersions.

The approach and solid-state form preferred will depend on the nature of the molecule and the targeted performance. However, APIs are more conveniently developed as oral-dosage forms if in the crystalline state.

Cocrystals became known as an alternative crystal-engineering platform to improve the physicochemical properties of challenging crystalline molecules. Although an emerging technology, cocrystals are now being considered as a potential alternative to amorphous drugs and amorphous solid dispersions, due to their improved overall stability and performance *(*Cryst. Growth Des. 2011, 11, 2662-79*).*

Pharmaceutical cocrystals are multicomponent crystals of, at least, two molecules (*e.g*. an API, or the salt of an API, and a coformer) to achieve a stable molecular complex *(*J. Am. Chem. Soc. 2004, 126, 13335-42*).* As there are no specific structural requirements for cocrystal formation, in opposition *e.g.* to salt formation where the molecule is required to have ionizable groups, cocrystallization can, in theory, be applied to any molecule (acids, bases, non-ionizable molecules). Examples of preferred APIs include, but not exclusively, the ones that are dissolution rate and solubility limited (*i.e.* BCS class II) *(*J. Am. Chem. Soc. 2003, 125, 8456-57*),* poorly stable *(*Int. J. Pharm. 2006, 320, 114-23*)*, hygroscopic *(*Cryst. Gowth Des. 2005, 5(3), 1013-21*)* or poorly compressible *(*Cryst. Growth Des. 2008, 8(5), 1575-79*).* The coformer used is an active or non-active ingredient (*e.g*. pharmaceutical excipients, vitamins, minerals, amino acids, other APIs) that has a favorable intermolecular interaction with the API, promoting hydrogen bonding, van der Wall forces or pi-stacking *(*US 2012/0258170 A1; US 2014/0031403A1). The resulting cocrystal presents very different physicochemical properties compared to the parent API, co-former or their physical mixture.

Other opportunities for cocrystals are related with permeability and bioavailability enhancement of APIs (*i.e*. BCS Class III and IV) *(*US 2011/0028435 A1*)*, improvement of down-stream processability (*e.g*. particle size, morphology, flowability), among other physicochemical properties (*e.g*. purity, toxicity, taste) *(*Pharmaceutical Salts and Co-crystals. 2012. 110-27*).* Moreover, cocrystallization can also be applied for the benefit of the production process itself. For example, cocrystallization has been used as a separation technique either for biological and chemical reactions. The formation of the cocrystal is promoted with one of the final products resultant from a fermentation, in order to facilitate product removal and increase process yield *(*Cryst Growth Des. 2010, 10 (3), 1171-79*).* In a second example, cocrystallization has been promoted to overcome the typical challenges in the blending steps, namely the elimination of batch to batch blend variability and particle segregation problems during product manipulation and transportation *(*US 2011/0028435 A1*).*

Despite the great potential of cocrystallization, producing cocrystals with high efficiency at industrial scales is challenging due to the tight thermodynamic and kinetic crystallization windows necessary to achieve the desired crystalline form.

The typical manufacturing methods used for the production of cocrystals include, but are not limited to, solution crystallization and mechanochemical methods. In solution-based methods, cocrystals are generally synthesized by dissolution and re-crystallization through slow evaporation *(*Pharm. Res. 2007 25(3), 530-41*)* or cooling *(*Cryst. Growth Des. 2010, 10(8), 3763-69*)* or by the addition of a liquid antisolvent *(*Eur. J. Pharm. Bio. 2013, 85, 854-61*).* In mechanochemical-based methods such as grinding, the solids are ground together under dry conditions [*i*.*e*. neat grinding *(*Pharm. Res. 2006, 23(10), 2381-92*)*] or with the presence of solvents [*i*.*e*. solvent-drop grinding *(*Cryst. Growth Des. 2009, 9(2), 1106-23*)*].

The use of these traditional techniques presents some challenges:
- Solution-based methods although amenable to reproducibly scale-up in stirred tanks they require relatively large volumes of organic solvents (environmentally undesirable) and a long time to obtain the reaction products. Moreover, there should be knowledge of the ternary phase diagram between the co-crystal constituents and the solvent, otherwise the risk of precipitation of pure compounds or the formation of other crystalline forms is high. Other issues include the fact that API and conformer(s) may undergo undesired interactions with organic solvents, which may be incorporated into the crystal lattice with the possibility of solvate/hydrate formation;
- The use of neat grinding or solvent-assisted grinding in cocrystallization is considered a more environmentally friendly process, however, more difficult to scale-up. Resultant cocrystals may also become electrostatically charged as a result from the stress applied during grinding. Grinding methods may also generate some degree of amorphization and/or crystal defects in the resultant products.

Recently, new methods have been developed in the field of pharmaceutical cocrystallization. These processes seek for the ease of scalability, continuous processing, robustness and cost-effectiveness while meeting the global trend to green and sustainable technologies. For example, Padrela *et al.* explored the implementation of supercritical CO₂-based methods for the screening and design of cocrystals. Authors showed that supercritical CO₂ can act as a solvent, anti-solvent or atomization enhancer *(*Eur. J. Pharm. Sci. 2009, 38, 9-17*)*. The possibility of obtaining cocrystals in a particulate form with different morphologies, shapes and particle sizes (from nano to micro) *(*J. of Supercritical Fluids. 2014, 86, 129-36*)* combined with the use of "green" solvents is presented as an advantage. Despite the promising results commercial scale apparatus are still not available mainly due to the high investment required *(*Scale-up issues for supercritical fluid processing in compliance with GMP. 2004, Chapter 15, Marcel Dekker, Inc.*)*.

Spray drying (SD) has also been evaluated for the preparation of "engineered" pharmaceutical cocrystals, because it also offers the possibility of manipulating the particle properties of cocrystals. Although typically used to prepare amorphous materials, theophylline-based cocrystals with optimized particle characteristics for inhalation purposes were successfully obtained by Alhalaweh et al. (AAPS PharmSciTech. 2013, 14(1), 265-76*).* Spray drying is a well-established technique for material processing and scale-up, although the limitations inherent to the use of solvents should not be neglected (*i.e*. process time, costs, environmental impact).

High Pressure Homogenization (HPH) and Twin-Screw Extrusion (TSE) are two of the most recent technologies used for continuous cocrystal formation *(*Cryst. Growth Des. 2013, 13, 2013-24*;* US 2010/013035 A1)*.* The use of homogenizers also enables the possibility to obtain a final product with the desired properties (*i.e*. particle size distribution) in a single process step, but one the other hand it implies the use of solvents. In opposition the extrusion is a solvent-free process, but with disadvantages associated with the downstream processing of the extrudates. Additional processing by milling, granulation or pelletization can lead to undesirable solid-state changes (*e.g*. amorphization, polymorphic transitions).

The new method herein described for the production of cocrystals is governed by similar principles of SD and TSE. It can be considered a hybrid technology and comprises the advantages of obtaining the final product in a particulate form, in a single stage operation without the need of any downstream processing. Moreover is a "green" and solvent-free process. This method can be conducted in the same apparatus of SD, with only minor modifications, thus making the commercial scale-up of the process readily available *(*Chimica Oggi - Chemistry Today. 2013, 31(5), 69-72*)*. The absence of solvents during the cocrystallization process complies with the "green" chemistry requirements, allows costs reduction and avoids the formation of hydrates or solvates. The new method herein described implies a melting step, which according to the physicochemical properties of the API and coformers, can occur at high temperatures and thus attention should be paid with heat labile compounds and degradation profiles.

The present invention discloses a scalable and solvent-free method to produce cocrystals. According to one aspect of the invention, there is provided a method of making cocrystals with at least a first substance and a second substance which are able to form cocrystals, wherein the first substance is an active pharmaceutical ingredient (API) or a pharmaceutically acceptable derivative, such as a salt, of an API, which method comprises the steps of:
a) feeding a molten mixture of the at least first substance and second substance into an atomizer;
b) atomizing the molten mixture to droplets;
c) solidifying the droplets to particles; and
d) collecting the said particles.

According to one aspect of the present disclosure, there is provided a method of making cocrystals, which method comprises the steps of:
a) feeding a molten mixture of at least a first substance and a second substance which are able to form cocrystals to an atomizer;
b) atomizing the molten mixture to droplets;
c) solidifying the droplets to particles;
d) collecting the said particles.

In particular, the method of the invention allows cocrystals to be obtained in the form of particles. Preferably, the method is carried out without the use of any solvents ie. it is solvent-free. Preferably, the method is carried out as a single stage operation. The method involves forming a molten mixture of at least two substances, although more than two substances may be used if desired. The at least two substances should be such that they are able to form cocrystals, particularly under the preferred process conditions applicable for the chosen substances. Preferably, the step of solidifying the droplets comprises cooling, and any suitable form of cooling may be used. Unexpectedly, the cooling of the high surface area droplets generates the cocrystal particles. The cooling conditions required are dependent on the system tested and its specific thermodynamic and kinetic requirements, as will be clear to those skilled in this particular art. The size and morphology of the cocrystal particles obtained can also be controlled by varying the process parameters. Particle-based compositions using particles made according to the invention are also disclosed in this invention.

Accordingly, the disclosure describes uses of cocrystals made according to the method of the present invention in the formulation of a pharmaceutical composition.

The disclosure also describes cocrystals obtainable or obtained by the method of the present invention. In particular, the disclosure describes cocrystals in the form of particles obtainable or obtained by the method of the invention. The particles are preferably made by controlled cooling.

The disclosure describes a pharmaceutical composition comprising cocrystals made according to the method of the invention. In particular, the disclosure describes a pharmaceutical composition comprising cocrystals in the form of particles made according to the method of the invention.

Comparing with the existing prior art, the advantages of the present invention include:
- The process conditions (*e.g*. melt temperature, cooling rate, feed flow, among others) can be tailored to achieve the desired crystalline form, fitting the thermodynamic/kinetic requirements for different cocrystal compositions;
- The cocrystals obtained are in particulate form, avoiding subsequent downstream steps that can lead to solid-state changes (*e.g*. amorphization during milling procedures);
- The shape and morphology of the particle can be controlled through process parameters, such as the viscosity of the melt, the type of atomizer used, the atomization ratio or pressure. Particle size and morphology are determining factors with respect to product performance (*e.g*. solubility, dissolution rate, absorption) and mechanical properties (*e.g*. compressibility, flow properties);
- Since small droplets (and not the bulk material) are cooled/solidified, a more uniform cooling of the material occurs due to the high surface area of the droplets. Such uniformity can influence the efficient conversion to the targeted crystalline form;
- The process is easily scalable and can be carried out using spray drying capabilities;
- No organic solvents are used, being a green chemistry and cost-effective process. No additional steps for solvent removal are required, which also avoids the formation of hydrates or solvates.

In step a, the molten mixture of at least a first substance and a second substance are suitably prepared by heating. Preferably, the substances are exposed to heat for at least 1 minute, preferably 2 minutes or longer, until a homogenous and molten mixture is formed. As will be understood, the length of time required to obtain the molten mixture and the mixture melting temperature generally depends on the materials used, their physicochemical properties (*e.g*. melting temperature) and degradation profile. Melting temperatures of organic compounds may vary between -120 to +300°C, typically between 0 and 200°C, especially between 20 and 180°C *(*Cryst. Growth Des. 2004, 4(5), 879-80*).* The temperature setpoint for melting the mixture composed of at least two substances should preferably be at a temperature around the melting point of the cocrystal constituent with the lowest melting point. If it is known that there is the potential to form a eutectic mixture, the setting temperature for melting the mixture should preferably be that of the eutectic point. It has been reported that cocrystals can be formed from the eutectic melt. Additional mechanical forces, such as shear and pressure, may be employed to facilitate the mixture and entanglement of the components in the mixture, as well as to decrease their viscosity. Examples of mixing elements include, but are not limited to, magnetic stirrer bars, paddles, and combining thermo-regulated single or twin screws with the apparatus feeding/atomization system. The use of single or twin screws or an extruder to form the molten mixture also contributes to a reduction of the time during which the at least two substances are exposed to the chosen temperature - relative to the conventional method that uses a heated beaker or reactor.

In a preferred aspect of the invention, the first substance may be an Active Pharmaceutical Ingredient (API) and the second substance may be a coformer. If desired, more than one API may be used, and/or more than one coformer may be used. The API can be mixed with the coformer to form the molten mixture, preferably in a molar stoichiometric ratio, such as 1:1, 1:2, 2:1 or another integer ratio. The API may in principle be any API, but is preferably one which has at least one functional group selected from: thioether, alcohol, thiol, aldehyde, ketone, thioketone, nitrate ester, phosphate ester, thiophosphate ester, ester, thioester, sulfate ester, carboxylic acid, phosphonic acid, phosphinic acid, sulfonic acid, amide, primary amine, secondary amine, ammonia, tertiary amine, imine, thiocyanate, cyanamide, oxime, nitrile, diazo, organohalide, nitro, S-heterocyclic ring, thiophene, N-heterocyclic ring, pyrrole, O-heterocyclic ring, furan, epoxide, peroxide, hydroxamic acid, imidazole, and pyridine. Examples of preferred APIs include, but are not limited to, active compounds with poor solubility and low dissolution rate (*i.e*. BCS class II) *(*J. Am. Chem. Soc. 2003, 125, 8456-57*),* compounds with poor stability *(*Int. J. Pharm. 2006, 320, 114-23*)*, compounds with high hygroscopicity *(*Cryst. Gowth Des. 2005, 5(3), 1013-21*)* or compounds with poor compressibility *(*Cryst. Growth Des. 2008, 8(5), 1575-79*).*

The coformer may in principle be any suitable coformer with respect to the first substance, which is preferably an API, but preferably the coformer has at least one functional group selected from: thioether, alcohol, thiol, aldehyde, ketone, thioketone, nitrate ester, phosphate ester, thiophosphate ester, ester, thioester, sulfate ester, carboxylic acid, phosphonic acid, phosphinic acid, sulfonic acid, amide, primary amine, secondary amine, ammonia, tertiary amine, imine, thiocyanate, cyanamide, oxime, nitrile, diazo, organohalide, nitro, S-heterocyclic ring, thiophene, N-heterocyclic ring, pyrrole, O-heterocyclic ring, furan, epoxide, peroxide, hydroxamic acid, imidazole, and pyridine. The coformer used may be an active or non-active ingredient (*e.g*. pharmaceutical excipients, vitamins, minerals, amino acids, other APIs) that has a favorable intermolecular interaction with the API, promoting hydrogen bonding, van der Wall forces or pi-stacking *(*US 2012/0258170 A1; US 2014/0031403 A1*).*

In addition to the first and second substances, which are preferably an API and a suitable coformer, the molten mixture may if desired also include additional functional matrix materials. The presence of these matrix materials is typically to achieve one or more of the following: promote a better mixing between API and coformer(s), decrease the melting temperature of the mixture, or adding functionality to the final product (*e.g*. improved performance, mechanical properties, among others). Examples of functional matrix materials may include, but are not limited to, agents with a plasticizing effect, ie. a plasticizer (*e.g.* polyethylene glycol, carbon dioxide), surfactants, or polymers *(*J. Pharm. Sci. 2014, May 7*).*

In step b, the atomization can in principle be done using any suitable technique for producing droplets from a molten mixture. For example, an apparatus comprising an atomizer nozzle may be used. Preferably, the atomization occurs at a nozzle. Preferably, the feeding of the molten mixture to the nozzle is performed through a thermo-regulated line to assure consistent temperatures. Preferably, the nozzle itself is also heated up (for example, by the circulation of heated thermal fluid). This can help to control the molten mixture temperature during atomization. The nozzle type used may for example include, but is not limited to: pressure nozzles, fluid nozzles, ultrasonic nozzles, or rotary atomizers. Other droplet-formation systems known by those skilled in the art may also be used.

In step c, the solidification of the droplets is preferably done by controlled cooling. The controlled cooling may comprise (to promote the cooling) a stream of co-current or counter-current cooling gas. This may for example be, but is not limited to, air, nitrogen or carbon dioxide. The temperature of the inlet gas is important in determining the cooling rate of the molten droplets, and therefore should be adjusted depending on the system tested. The temperature of the inlet gas may, for example, range from -20 to 200°C. Chamber dimensions are also important to assure enough residence time of the droplet for a controlled cooling. The inner chamber volume can, for example, be below 50 m³, but preferably it is below about 10 m³. For materials requiring slower cooling rates to achieve the targeted crystalline form, a "fluidized spray congealing" set up may be applied, using a counter-current stream of gas with the required temperature and flow rate. Chamber-free concepts are also feasible.

In step d, the particles obtained preferably have a typical particle size from 1 to 500 µm. Particle recovery may be performed through a number of different suitable approaches. Examples include recovery in the cooling chamber, or after a cyclone. Those skilled in the art will be aware of other particle-recovery systems. In a preferred aspect of the the method of the, particles comprising at least 50% (w/w) cocrystal purity, more preferably at least 75% (w/w) cocrystal purity, especially 90% (w/w) or more cocrystal purity are obtained. The determination of the purity of the cocrystal product obtained is related to the quantification of its crystalline precursors that were not converted to cocrystals. These pure components are considered as impurities and can impact the overall performance of the final cocrystal product (*e.g*. solubility, bioavailability, stability, compressibility, among others). Analytical techniques for the quantification of cocrystal purity include, but are not limited to, Fourier-transformed infrared (FTIR), near-infrared (NIR), Raman, X-ray powder diffraction (XRPD), differential scanning calorimetry (DSC), high performance liquid chromatography (HPLC) *(*J. Pharm. Bio. Ana. 2014, 89, 166-175*;* Drug Dev. Ind. Pharm. 2012, 38(8), 923-929*;* J. Supercritical Fluids. 2013, 83, 78-85*).*

If desired, steps a to c may be run as batch processing or continuous processing.

Optionally, particularly for systems difficult to cocrystallize, or where the conversion to the cocrystal form is not sufficient during the cooling step, further downstream processing may be performed or may be required. This may, for example, comprise exposure of the particles to controlled conditions of temperature and humidity to favor the conversion to the suitable crystalline form. Downstream processing of the particles may also be performed if desired, and is encompassed within the scope of this invention.

Particles obtainable or obtained by the method of the invention can be further formulated into compositions, for example into a pharmaceutical composition, which may be used in the treatment, prevention or diagnosis of medical conditions. The disclosure thus relates to a composition, in particular a pharmaceutical composition, comprising cocrystal particles obtainable or obtained by the method of the present invention, and one or more suitable excipients, for example one or more pharmaceutically acceptable excipients. Examples of typical post-formulation excipients include, but are not limited to , density agents (*e.g*. lactose, microcrystalline cellulose, among others), binders (*e.g*. starch, celluloses, polyvinyl pyrollidone, among others), disintegrants (sodium starch glycollate, crosslinked polyvinyl pyrollidone, among others), and wetting agents. Examples of pharmaceutical forms for administration of cocrystals synthesized through the method herein described may include, by way of example only, solid dosage forms such as tablets, capsules, granules, pellets or powders.

Preferably, the apparatus used in steps b to d comprises a spray congealing (also referred as spray cooling, spray chilling or melt congeal) set up. This is defined as a process by which a liquid melt is atomized into a spray of fine droplets of spherical shape inside a cooling chamber. Here, the droplets meet an airstream sufficiently cold to solidify the droplets. The transition of a melt from a soft or fluid state to a rigid or solid state by cooling is called congealing. Previous studies have shown the use of spray congealing for the preparation of microparticles with the objective of increasing the solubility and dissolution rate of APIs with poor water solubility *(*Int. J. Pharm. 2009, 381(2), 176-83*),* obtaining controlled-release dosage forms *(*Eur. J. Pharm. Bio. 2008, 70, 409-20*)* and taste masking applications *(*Chem. Pharm. Bull. 1999, 47(2), 220-25*).* Spray congealing can be performed at different scales (from lab to industrial) with high efficiency.

For a better understanding of the present invention and in order to demonstrate the utility of spray congealing in cocrystal formation, three examples of the present invention include: two model APIs (*i.e.* caffeine and carbamazepine) and three suitable coformers (*i.e*. glutaric acid, salicylic acid and nicotinamide). Both the APIs and the coformers selected are typical model systems reported in literature. The "model" cocrystals used as examples for this invention are listed in Table 1, together with the reference to scientific papers that have also explored the cocrystallization of these same systems.

**Table 1. Reported cocrystal systems that were used as examples of embodiments of the present invention.**

| **API/Coformer System** | **Molar Proportion** | **Processing method** | **Reference*** |
|---|---|---|---|
| Caffeine/Glutaric Acid (CAF/GLU AC) | 1:1 | Solvent-drop grinding Solid state grinding | Trask *et al.* (2004) |
| | | | Lu *et al.* (2008) |
| | | | |
| Caffeine/Salicylic Acid (CAF/SAL AC) | 1:1 | Slurry method | Lu *et al.* (2008) |
| Carbamazepine/Nicotinamide (CARB/NIC) | 1:1 | Slurry method Solid state grinding Melting method | Lu *et al.* (2008) |
| | | | Chieng *et al.* (2009) |
| | | | Liu *et al.* (2012) |

| | | | |
|---|---|---|---|
| * Trask et al. Chem. Commun. 2004, 7, 890-91*;* Lu et al. Cryst. Eng. Comm. 2008, 10, 665-68*;* Chieng et al. Cryst. Growth Des. 2009, 9(5). 2377-86*;* Liu et al. Pharm. Res. 2012, 29, 806-17*.* | | | |

Reference to the accompanying figures, detailed description of process conditions and results obtained are set forth in the sections below.

### DESCRIPTION OF FIGURES

The present invention is further illustrated below, by the way of examples, with reference to the accompanying figures, in which:
FIG.1 shows the mDSC heat flow curves correspondent to the thermal analysis of pure CAF, pure GLU AC, CAF:GLU AC physical mixture (1:1) and CAF:GLU AC (congealed) powder (1:1).
FIG.2 shows the XRPD patterns correspondent to the pure CAF, pure GLU AC, CAF:GLU AC physical mixture (1:1) and CAF:GLU AC (congealed) powder (1:1).
FIG.3a to 3c shows the SEM micrographs correspondent to CAF:GLU AC (congealed) powder (1:1) at 100x, 350x and 650x magnification, respectively.
FIG.4 shows the mDSC heat flow curves correspondent to the thermal analysis of pure CAF, pure SAL AC, CAF:SAL AC physical mixture (1:1) and CAF:SAL AC (congealed) powder (1:1).
FIG.5 shows the XRPD patterns correspondent to the pure CAF, pure SAL AC, CAF:SAL AC physical mixture (1:1) and CAF:SAL AC (congealed) powder (1:1).
FIG.6a to 6c shows the SEM micrographs correspondent to CAF:SAL AC (congealed) powder (1:1) at 500x, 1000x e 5000x magnification, respectively.
FIG.7 shows the mDSC heat flow curves correspondent to the thermal analysis of pure CARB, pure NIC, CARB:NIC AC physical mixture (1:1) and CARB:NIC (congealed) powder (1:1).
FIG.8 shows the XRPD patterns correspondent to the pure CARB, pure NIC, CARB:NIC physical mixture (1:1) and CARB:NIC (congealed) powder (1:1).
FIG.9a and 9b shows the SEM micrographs correspondent to CARB:NIC (congealed) powder (1:1) at 300x, 650x, respectively.

Suitable examples are described below. These are intended to illustrate the present invention, and do not limit the scope thereof.

### EXAMPLE 1

A mixture of caffeine (CAF, β-caffeine anhydrous from Sigma-Aldrich Company,Ltd) and glutaric acid (GLU AC, from Sigma-Aldrich Company,Ltd), in a molar proportion 1:1 (total mass of ∼50 grams), was physically blended in a Turbula mixer for 10min at 46rpm. The physical mixture was slowly fed into a jacketed beaker with a magnetic stirrer inside. A thermal fluid, which is heated on a thermostatic bath, circulates inside the jacket of the beaker, feed line, and nozzle in order to keep the mixture in a molten state until atomized. The temperature set point of the thermostatic bath was initially set to a temperature around the melting point of the cocrystal constituent with the lowest melting point. Then, the temperature was increased, through smaller temperature increments, until total melting of both API and coformer was observed (*i.e*., 140°C).

Cocrystals were produced using a lab scale spray dryer (4M8-TriX ProCepT, Zelzate, Belgium), adapted for spray congealing and operated in open cycle mode (*i.e*. without recirculation of the congealing nitrogen). The cooling chamber height was set to its maximum (extended set-up ∼197cm). Atomization was conducted with a jacketed two fluid nozzle (tip of 1.20 mm and respective cap) that was used to atomize the melt. A flow rate of 11 L/min was set for the atomization with nitrogen. Co-current nitrogen was used to promote the solidification of the melt after atomization. The congealing gas flow rate was set to 0.35 m³/min.

Before feeding the melt to the nozzle, the spray congealing unit was stabilized with nitrogen to assure stable inlet (T_in) and outlet (T_out) temperatures (*i.e*., 140°C and 75°C). After stabilization, the melt was fed by pressurizing the beaker using a pressure regulator for the liquid feed rate. The droplets were then cooled in the spray-congealing chamber by the co-current nitrogen. The stream containing the product was directed to a cyclone to separate the solids from the gas.

At the end of the process, the powders were cooled and analyzed by modulated differential scanning calorimetry (mDSC), X-ray powder diffraction (XRPD) and scanning electron microscopy (SEM) for characterization. The pure API, coformer and respective physical mixture (same molar proportion) were also analyzed through mDSC and XRPD for comparison.

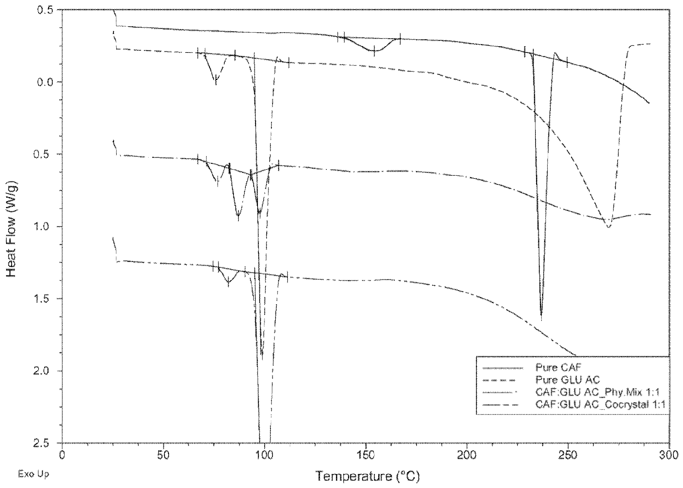

FIG.1 shows the mDSC total heat flow curves correspondent to the thermal analysis of pure CAF, pure GLU AC, 1:1 CAF:GLU AC physical mixture and 1:1 CAF:GLU AC powder obtained by spray congealing. mDSC experiments were performed in a TA Q1000 (TA Instruments, New Castle, Delaware, USA) equipped with a Refrigerated Cooling System (RCS). The enthalpy response was calibrated using indium. Samples weighing between 5-10 mg were analyzed in pinholed DSC aluminum pans and under continuous dry nitrogen purge (50 mL/min). Samples were analyzed using a modulated heating ramp from 25°C to 300°C at a heating rate of 5°C/min using a period of 60s and amplitude of 0.8°C. Data was analyzed and processed using the TA Universal Analysis 2000 Software.

The DSC profile correspondent to the pure CAF presented two endothermic peaks, one at 140°C and the other at 233°C. According to Pinto *et al.* the first peak corresponded to the transition of β-caffeine (or form II) to α-caffeine (or form I) and the second peak corresponded to the formation of an isotropic liquid when heating the α-anhydrous form *(*J. Chem. Thermodynamics. 2006, 38, 1515-22*).* Similarly, the DSC profile of pure GLU AC also presented two endothermic peaks (*i.e*. 70°C and 95°C, respectively), in which the first one is related to a solid-solid transformation, and the second peak to the melting of the new solid-phase formed *(*Pharm. Res. 2006, 23(8), 1888-97*).* In the thermogram of the 1:1 CAF:GLU AC physical mixture, the first peak at 71°C may correspond to the phase transformation of pure GLU AC, the second at 83°C corresponded to the melting of the CAF:GLU AC eutectic, and the third peak at 94°C to the melting of the cocrystal formed *(*Cryst. Eng. Comm. 2008, 10, 665-68*).*

The observation of a eutectic followed by cocrystal melting supports the cocrystal formation. More than 50% of the organic binary mixtures present a eutectic point and it is reported that cocrystals can be formed from the eutectic melt *(*Cryst. Growth Des. 2004, 4(5), 879-80*;* Cryst. Growth Des. 2009, 9(3), 1621-37*).* Among the different screening methods that have been developed to predict cocrystal formation, thermal methods like hot-stage microscopy (often referred as the Kofler method) and DSC have been used to assess if a particular API is able to cocrystallize with a coformer using the melt/eutectic information *(*Cryst. Growth Des. 2008, 8(5), 1697-12*;* Cryst. Eng. Comm. 2008, 10, 665-68*).*

Comparing the thermogram of the 1:1 CAF:GLU AC physical mixture with the 1:1 CAF:GLU AC crystalline material produced using the method presented in this invention, it can be observed that the peak correspondent to the phase transition of pure GLU AC disappeared, while the eutectic melting and the cocrystal melting peaks remained. Moreover the temperatures obtained in both cases are also in agreement. These results are indicative of 1:1 CAF:GLU AC cocrystal formation using spray congealing.

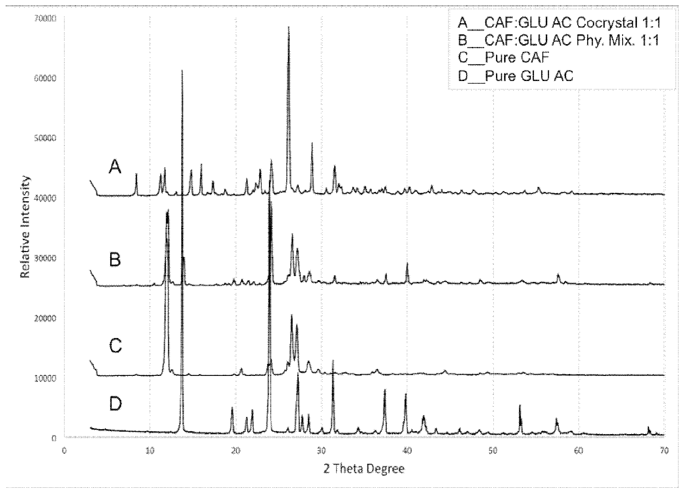

XRPD analyses were conducted in order to further characterize the material. FIG.2 shows the XRPD patterns correspondent to the pure CAF, pure GLU AC, 1:1 CAF:GLU AC physical mixture and 1:1 CAF:GLU AC powder obtained using spray congealing. XRPD experiments were performed in a D8 Advance Bruker AXS Theta-2Theta diffractometer with a copper radiation source (Cu Kalpha2, wavelength = 1.5406 Å), voltage 40 kV, and filament emission 35 mA. The samples were measured over a 2θ interval from 3 to 70° with a step size of 0.017° and step time of 50 s.

The XRPD diffractogram obtained with 1:1 CAF:GLU AC physical mixture was equivalent to the pure crystalline starting components (CAF and GLU AC). Moreover, the XRPD patterns obtained for the pure components are equivalent to the patterns found in the literature for the same compounds *(*J. Chem. Soc. 1997*,* Perkin Trans. 2. 1985-90*;* Pharm. Res. 2006, 23(8), 1888-97*).* By opposition, in the diffractogram of the spray congealed material new peaks (not characteristic of CAF or GLU AC) were detected. These were equivalents to the diffractograms of CAF:GLU AC 1:1 cocrystals available in the literature *(*Chem. Commun. 2004, 7, 890-91*;* Cryst. Growth Des. 2005, 5(3), 1013-21*).* Discriminative cocrystal peaks include the ones at: 8.4° 2θ, 11.2° 2θ, 11.7° 2θ, 14.8° 2θ, 16.0° 2θ, 17.4° 2θ, 22.3° 2θ, 22.8° 2θ, 26.2° 2θ and 28.9° 2θ.

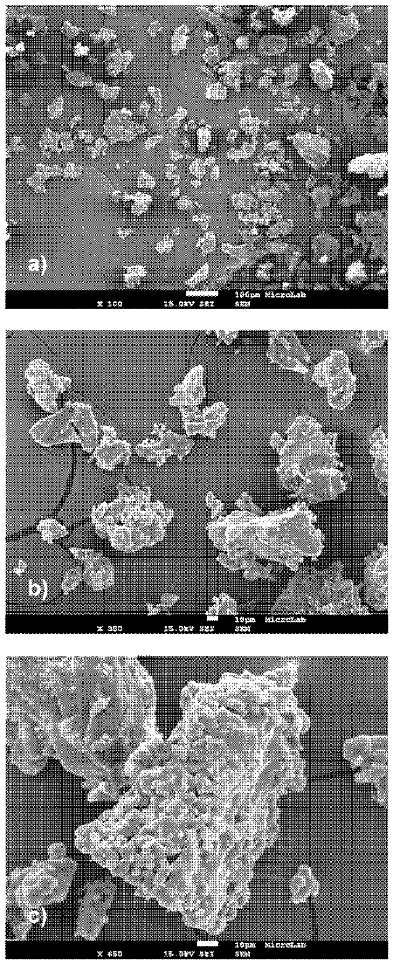

FIG.3a to 3c shows the SEM micrographs correspondent to 1:1 CAF:GLU AC powder produced by spray congealing at 100x, 350x and 650x magnification, respectively. During analysis the samples were attached to adhesive carbon tapes (Ted Pella Inc., CA, USA), previously fixed to aluminum stubs where the powder in excess was removed by a jet of pressurized air. The samples were left in vacuum for 2 hours and then were coated with gold/palladium (South Bay Technologies, model E5100, San Clement, CA). A JEOL JSM-7001F/Oxford INCA Energy 250/HKL scanning electron microscope (JEOL, Japan) in high vacuum operated at a typical accelerating voltage of 15 - 20kV.

Analyzing FIG.3 large agglomerated particles were obtained, varying in a size range of 50 to 100 µm. Few agglomerates featured a spherical shape, while the majority presented a more irregular/prismatic shape. Observation of cocrystal agglomerate surface under high magnification revealed that the agglomerates consisted of rod- and plate-shaped individual cocrystals entangled or fused with each other to give spherical/prismatic agglomerates (FIG. 3c).

### EXAMPLE 2

Example 2 of the present invention consists in a mixture of caffeine (CAF, β-caffeine anhydrous from Sigma-Aldrich Company,Ltd) and salicylic acid (SAL AC, from Sigma-Aldrich Company,Ltd), in a molar proportion 1:1 (total mass of ∼30 grams). Further processing of the physical mixture was identical to the procedure applied in EXAMPLE 1. Total melting of the physical mixture was observed at 150°C. Spray congealing was conducted in the same equipment and in the same conditions as in EXAMPLE 1 (*i.e*. open-cycle mode, extended set-up, jacketed two fluid nozzle with 1.20 mm tip). The atomization flow rate was set to 9.2 L/min, while the congealing gas flow rate was maintained to 0.35 m³/min. The inlet and outlet temperatures (*i.e*. T_in and T_out, respectively) of the congealing gas after stabilization were 100°C and 58°C. At the end of the process, the resultant powders were characterized by the same analytical techniques and respective experimental methods as in EXAMPLE 1 (i.e. mDSC, XRPD and SEM) for characterization. The pure coformer and respective physical mixture were also analyzed through mDSC and XRPD for comparison.

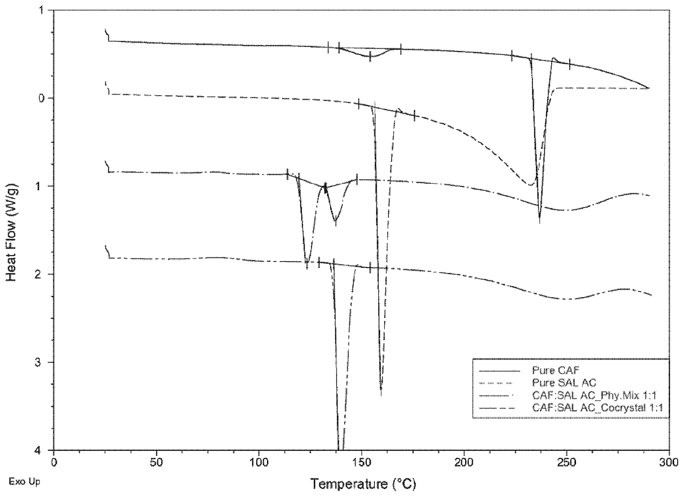

FIG.4 shows the mDSC heat flow curves correspondent to the thermal analysis of pure CAF, pure SAL AC, CAF:SAL AC physical mixture (1:1) and CAF:SAL AC (congealed) powder (1:1).

The interpretation of the DSC profile correspondent to the pure CAF is the same as the one described in EXAMPLE 1. The DSC profile of pure SAL AC presented a sharp endothermic peak at 156°C attributed to the melting of the material followed by a broad endothermic peak that corresponding to degradation. The observed endothermic peaks in the thermogram of the 1:1 CAF:SAL AC physical mixture are equivalent to the ones observed by Lu et al. (Cryst. Eng. Comm. 2008, 10, 665-68*).* The first peak at 119°C and the second peak at 133°C corresponded to the eutectic and cocrystal melting respectively. Again, with the preliminary DSC analysis of the 1:1 CAF:SAL AC physical mixture it is possible to infer about the potential of cocrystal formation.

Comparing the thermogram of the 1:1 CAF:SAL AC physical mixture with the 1:1 CAF:SAL AC crystalline material produced using spray congealing, it can be observed that the peak correspondent to the eutectic disappeared, while the cocrystal melting peak remained within the same temperature range (*i.e*. 136°C). These results are indicative that high purity 1:1 CAF:SAL AC cocrystals were formed. The same cocrystal system prepared by the slurry method also presented a single sharp endotherm at ∼140°C attributed to the cocrystal melting *(*Cryst. Eng. Comm. 2008, 10, 665-68*)*.

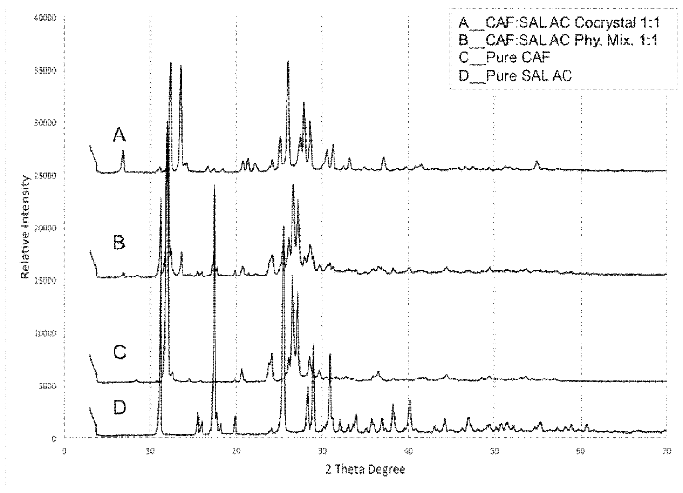

FIG.5 shows the XRPD patterns correspondent to the pure CAF, pure SAL AC, 1:1 CAF:SAL AC physical mixture and 1:1 CAF:SAL AC powder obtained using spray congealing.

The XRPD diffractogram obtained for the 1:1 CAF:SAL AC physical mixture was equivalent to the pure crystalline starting components (CAF and SAL AC). Moreover, the XRPD patterns obtained for the pure components are equivalent to the patterns shown obtained by Lu et al. (Cryst. Eng. Comm. 2008, 10, 665-68*).*

The diffractogram of the 1:1 CAF:SAL AC spray congealed material was different from that of pure crystalline CAF and SAL AC and similar to the simulated XRPD pattern obtained by Lu et al. (Cryst. Eng. Comm. 2008, 10, 665-68*).* These results indicate that another caffeine-based cocrystal was obtained through this innovative method. Discriminative cocrystal peaks include the ones at: 6.9° 2θ, 12.4° 2θ, 13.6° 2θ, 25.1° 2θ, 26.0° 2θ, 27.4° 2θ, 27.9° 2θ, 28.6° 2θ.

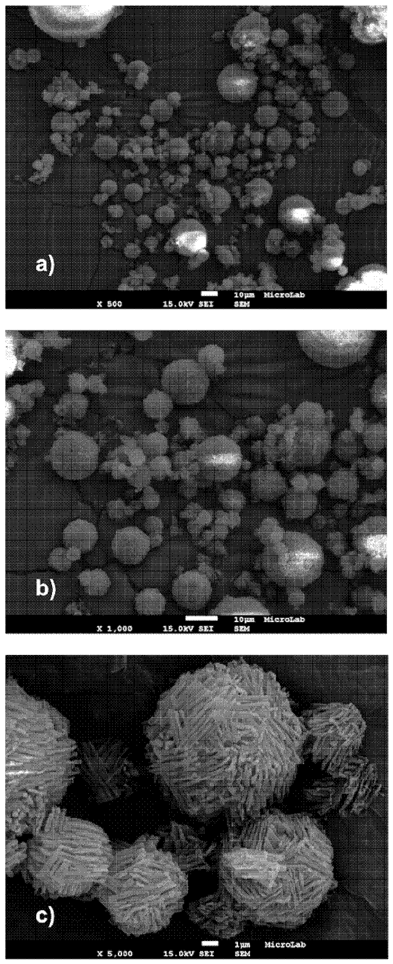

FIG.6a to 6c shows the SEM micrographs correspondent to 1:1 CAF:SAL AC powder obtained by spray congealing at 500x, 1000x and 5000x magnification, respectively.

Analyzing FIG.6 spherical agglomerated particles were obtained with an average particle size between 10 µm and 20µm. Observation of cocrystal agglomerate surface under high magnification revealed that the agglomerates consisted of elongated (tubular or acicular) shaped individual cocrystals cohesively bonded together (FIG. 6c). The crystal habit of individual cocrystals obtained from the same API may differ due to the inherent crystalline properties of the coformer.

### EXAMPLE 3

Example 3 of the present invention consists in a mixture of carbamazepine (CARB anhydrous from TCI Development Co., Ltd.) and nicotinamide (NIC, from Sigma-Aldrich Company,Ltd), in a molar proportion 1:1 (total mass of ∼30 grams). Further processing of the physical mixture was identical to the procedure applied in EXAMPLE 1 and 2. Total melting of the physical mixture was observed at 175°C. Spray congealing was conducted in the same equipment and in the same conditions as in EXAMPLE 1 and 2 (*i.e*. open-cycle mode, extended set-up, jacketed two fluid nozzle with 1.20 mm tip). The atomization flow rate was set to 11.8 L/min, while the congealing gas flow rate was maintained to 0.35 m³/min. The inlet and outlet temperatures (*i.e*. T_in and T_out, respectively) of the congealing gas after stabilization were 50°C and 36°C. At the end of the process, the resultant powders were characterized by the same analytical techniques and respective experimental methods as in EXAMPLE 1 and 2 (i.e. mDSC, XRPD and SEM) for characterization. The pure API, coformer and respective physical mixture were also analyzed through mDSC and XRPD for comparison.

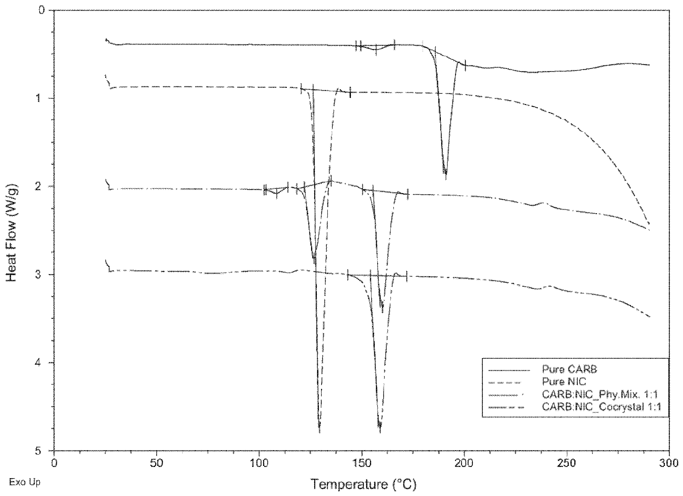

FIG.7 shows the mDSC heat flow curves correspondent to the thermal analysis of pure CARB, pure NIC, 1:1 CARB:NIC AC physical mixture and 1:1 CARB:NIC powder obtained using spray congealing.

Similarly to the DSC profile of pure CAF, pure CARB first underwent a polymorphic transformation at 150°C and second the melting of the new phase formed at 186°C *(*J. Pharm. Sci. 2003, 92(11), 2260-71*).* The thermogram of pure NIC presented a single endothermic peak at 126°C attributed to the thermodynamic melting of the material. The thermogram of the 1:1 CARB:NIC physical mixture showed two sharp endothermic peaks at 122°C and 157°C, which corresponded to the eutectic and cocrystal melting, respectively. Another endothermic peak was observed at a temperature of 103°C with a much smaller associated enthalpy as compared with aforementioned endothermic peaks, which may correspond to a phase transformation. The thermogram observed for the powder produced using spray congealing was in agreement with the thermogram of the respective physical mixture and was indicative that cocrystals were formed. Similarly to EXAMPLE 2, the endotherm correspondent to the eutectic disappeared while the peak correspondent to the melting of the cocrystal was still observed at 154°C. These results were in agreement with the literature, where 1:1 CARB:NIC cocrystals were produced using different manufacturing approaches *(*Cryst. Growth Des. 2009, 9(5). 2377-86*;* Pharm. Res. 2012, 29, 806-17*).*

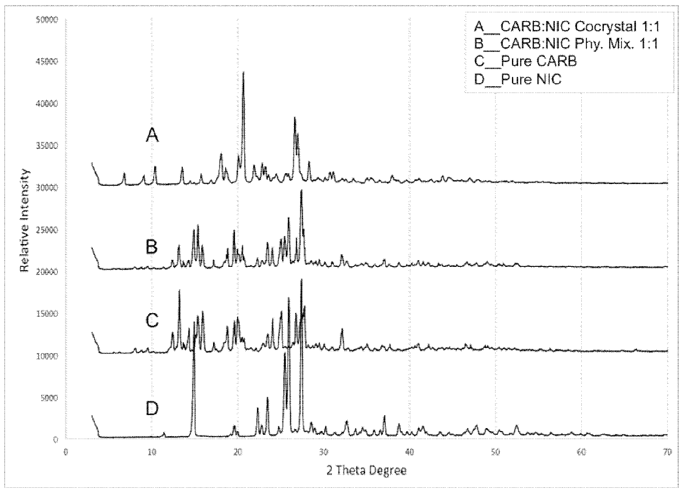

FIG.8 shows the XRPD patterns correspondent to the pure CARB, pure NIC, 1:1 CARB:NIC physical mixture and 1:1 CARB:NIC powder produced by spray congealing.

The XRPD patterns obtained for the pure components are equivalent to the patterns of CARB form III and NIC shown by Cheing et al. (Cryst. Growth Des. 2009, 9(5). 2377-86*).* In addition, the diffractogram obtained for the 1:1 CARB:NIC physical mixture was, as expected, equivalent to the patters of the pure crystalline starting components (CARB and NIC). Comparing the latter results with the XRPD diffractogram of the material produced by spray congealing it can be observed the appearance of new crystalline peaks and the overall decrease in the peak intensities of the characteristic peaks of the pure components. This XRPD 1:1 CARB:NIC cocrystal pattern is comparable to the cocrystal diffractogram obtained by Chieng *et al.,* who produced the same cocrystal by a solid-state grinding method *(*Cryst. Growth Des. 2009, 9(5), 2377-86*).* Discriminative cocrystal peaks include the ones at: 6.8° 2θ, 9.1° 2θ, 10.4° 2θ, 13.5° 2θ, 15.8° 2θ, 18.1° 2θ, 20.6° 2θ, 26.6° 2θ.

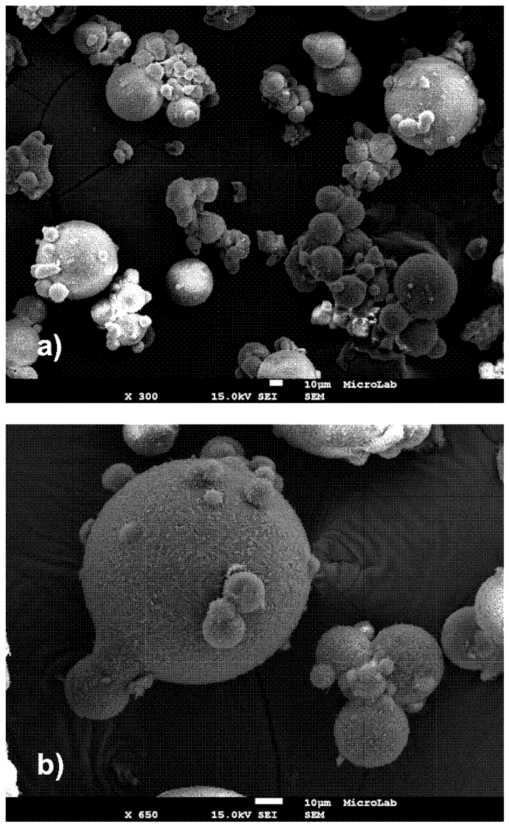

FIG.9a and 9b shows the SEM micrographs correspondent to 1:1 CARB:NIC final product obtained by spray congealing at 300x, 650x magnification, respectively.

Analyzing FIG.9, and similarly with FIG.6 with respect to the 1:1 CAF:SAL AC cocrystals, spherical agglomerated particles were obtained. In general, these particles present a particle size between 10µm and 50µm. Observation of cocrystal agglomerate surface under high magnification revealed that the agglomerates consisted of individual needle-shape cocrystals fused with each other.

## Claims

1. A method of making cocrystals with at least a first substance and a second substance which are able to form cocrystals, wherein the first substance is an active pharmaceutical ingredient (API) or a pharmaceutically acceptable derivative, such as a salt, of an API, which method comprises the steps of:
a) feeding a molten mixture of the at least first substance and second substance into an atomizer;
b) atomizing the molten mixture to droplets;
c) solidifying the droplets to particles; and
d) collecting the said particles.

2. A method according to claim 1, further comprising a following feature:
i) wherein the method is a batch process or continuous process;
ii) wherein the first and second substances are combined in a stoichiometric ratio, such as 1:1, 1:2, 2:1 or another integer ratio;
iii) wherein the method is a method of making pharmaceutical co-crystals; or
iv) wherein the first substance or its pharmaceutically acceptable derivative has at least one functional group selected from: thioether, alcohol, thiol, aldehyde, ketone, thioketone, nitrate ester, phosphate ester, thiophosphate ester, ester, thioester, sulfate ester, carboxylic acid, phosphonic acid, phosphinic acid, sulfonic acid, amide, primary amine, secondary amine, ammonia, tertiary amine, imine, thiocyanate, cyanamide, oxime, nitrile, diazo, organohalide, nitro, S-heterocyclic ring, thiophene, N-heterocyclic ring, pyrrole, O-heterocyclic ring, furan, epoxide, peroxide, hydroxamic acid, imidazole, and pyridine.

3. A method according to any preceding claim, wherein the second substance is a coformer, optionally wherein the coformer is an active or non-active ingredient.

4. A method according to claim 3, wherein the coformer is a pharmaceutical excipient, vitamin, mineral or amino acid.

5. A method according to any one of claims 3 to 4, wherein the coformer has at least one functional group selected from: thioether, alcohol, thiol, aldehyde, ketone, thioketone, nitrate ester, phosphate ester, thiophosphate ester, ester, thioester, sulfate ester, carboxylic acid, phosphonic acid, phosphinic acid, sulfonic acid, amide, primary amine, secondary amine, ammonia, tertiary amine, imine, thiocyanate, cyanamide, oxime, nitrile, diazo, organohalide, nitro, S-heterocyclic ring, thiophene, N-heterocyclic ring, pyrrole, O-heterocyclic ring, furan, epoxide, peroxide, hydroxamic acid, imidazole, and pyridine.

6. A method according to any preceding claim, wherein the first and second substances are exposed to heat for at least 1 minute, optionally 2 minutes or longer, until a homogenous and molten mixture is formed.

7. A method according to any preceding claim, wherein shear and/or pressure are employed to facilitate the mixture and entanglement of the components in the mixture, as well as to decrease their viscosity, optionally wherein shear and pressure are promoted by magnetic stirrer bars, paddles, or by an extrusion method.

8. A method according to claim 7, wherein the extrusion method is a screw-based extrusion apparatus connected to the atomization system.

9. A method according to claim 8, wherein the screw-based extrusion apparatus connected to the atomization system is a single-screw or twin-screw extrusion apparatus, optionally wherein the twin-screw extrusion apparatus is a co-rotating or counter-rotating system.

10. A method according to any preceding claim, further comprising one or more of the following :
i) wherein the melting temperature of the mixture is between -120 to +300°C, typically between 0 and 200°C, optionally between 20 and 180°C;
ii) wherein the mixture further comprises one or more functional matrix material(s) to promote mixing between the first and second substances, and/or to decrease the melting temperature of the mixture, and/or to add functionality to the final product, optionally wherein the one or more functional matrix material(s) comprises an, agent with a plasticizing effect, such as polyethylene glycol, or carbon dioxide, or one or more surfactant, or one or more polymers; and
iii) wherein in step b) the atomizing nozzle has a rotary, pressure, fluid, or ultrasonic configuration.

11. A method according to any preceding claim, wherein in step c) cooling is promoted by a stream of co-current or counter-current cooling gas or liquid, with respect to the melt spray direction.

12. A method according to any preceding claim, wherein the residence time of the droplets is controlled through a counter-current stream of cooling gas or liquid.

13. A method according to any one of claims 11 or 12, further comprising one or more of the following:
(i) wherein the initial temperature of the stream of gas or liquid is between -20 and 200°C;
(ii) wherein the counter-current stream of cooling gas is air, nitrogen or carbon dioxide;
(iii) wherein the counter-current stream of cooling liquid is liquid carbon dioxide or liquid nitrogen.

14. A method according to any one of claims 11, 12 or 13(i), further comprising one or more of the following:
(i) wherein the cooling gas is air, nitrogen or carbon dioxide;
(ii) wherein the cooling liquid is liquid carbon dioxide or liquid nitrogen.

15. A method according to any preceding claim, further comprising one or more of the following:
(i) wherein the particles obtained comprise at least 50% (w/w) cocrystal purity, more preferably at least 75% (w/w) cocrystal purity, especially 90% (w/w) or more cocrystal purity;
(ii) wherein the particles obtained have a particle size from 1 to 500 µm.

## Patentansprüche

1. Verfahren zur Herstellung von Co-Kristallen mit mindestens einer ersten Substanz und einer zweiten Substanz, die Co-Kristalle bilden können, wobei die erste Substanz ein pharmazeutischer Wirkstoff (API) oder ein pharmazeutisch zulässiges Derivat, wie ein Salz, eines API ist, wobei das Verfahren die folgenden Schritte umfasst:
a) Füllen einer geschmolzenen Mischung mindestens entweder der ersten Substanz oder der zweiten Substanz in einen Zerstäuber;
b) Zerstäuben dieser geschmolzenen Mischung zu Tröpfchen;
c) Verfestigen der Tröpfchen zu Partikeln; und
d) Auffangen der besagten Partikel.

2. Verfahren nach Anspruch 1, ferner ein folgendes Merkmal umfassend:
i) wobei das Verfahren ein diskontinuierliches oder kontinuierliches Verfahren ist;
ii) wobei die erste und zweite Substanz in einem stöchiometrischen Verhältnis, wie 1:1, 1:2, 2:1 oder einem anderen ganzzahligen Verhältnis, kombiniert werden;
iii) wobei das Verfahren ein Verfahren für die Herstellung pharmazeutischer Co-Kristalle ist; oder
iv) wobei die erste Substanz oder ihr pharmazeutisch zulässiges Derivat mindestens über eine Funktionsgruppe verfügt, ausgewählt aus: Thioether, Alkohol, Thiol, Aldehyd, Keton, Thioketon, Nitratester, Phosphatester, Thiophosphatester, Ester, Thioester, Sulfatester, Carbonsäure, Phosphonsäure, Phosphinsäure, Sulfonsäure, Amid, primärem Amin, sekundärem Amin, Ammoniak, tertiärem Amin, Imin, Thiocyanat, Cyanamid, Oxim, Nitril, Diazo, Organohalid, Nitro, S-heterozyklischem Ring, Thiophen, N-heterozyklischem Ring, Pyrrol, O-heterozyklischem Ring, Furan, Epoxid, Peroxid, Hydroxamsäure, Imidazol und Pyridin.

3. Verfahren nach einem der vorhergehenden Ansprüche, wobei die zweite Substanz ein Co-Former ist, wobei optional der Co-Former ein aktiver oder inaktiver Wirkstoff ist.

4. Verfahren nach Anspruch 3, wobei der Co-Former ein pharmazeutischer Trägerstoff, ein Vitamin, Mineral oder eine Aminosäure ist.

5. Verfahren nach einem der Ansprüche 3 bis 4, wobei der Co-Former mindestens über eine Funktionsgruppe verfügt, ausgewählt aus: Thioether, Alkohol, Thiol, Aldehyd, Keton, Thioketon, Nitratester, Phosphatester, Thiophosphatester, Ester, Thioester, Sulfatester, Carbonsäure, Phosphonsäure, Phosphinsäure, Sulfonsäure, Amid, primärem Amin, sekundärem Amin, Ammoniak, tertiärem Amin, Imin, Thiocyanat, Cyanamid, Oxim, Nitril, Diazo, Organohalid, Nitro, S-heterozyklischem Ring, Thiophen, N-heterozyklischem Ring, Pyrrol, O-heterozyklischem Ring, Furan, Epoxid, Peroxid, Hydroxamsäure, Imidazol und Pyridin.

6. Verfahren nach einem der vorhergehenden Ansprüche, wobei die erste und zweite Substanz für mindestens 1 Minute Hitze ausgesetzt werden, optional 2 Minuten oder länger, bis ein homogenes und geschmolzenes Gemisch entstanden ist.

7. Verfahren nach einem der vorhergehenden Ansprüche, wobei Schubspannung und/oder Druck angewendet wird, um das Mischen und Verknüpfen der Komponenten im Gemisch zu erleichtern, ebenso um deren Viskosität zu verringern, optional wobei die Schubspannung und der Druck durch Magnetrührstäbchen, Paddelrührer oder durch ein Extrusionsverfahren gefördert werden.

8. Verfahren nach Anspruch 7, wobei das Extrusionsverfahren eine schneckenbasierte Extrusionsvorrichtung darstellt, die mit dem Zerstäubersystem verbunden ist.

9. Verfahren nach Anspruch 8, wobei die schneckenbasierte Extrusionsvorrichtung, die mit dem Zerstäubersystem verbunden ist, eine Einzelschnecken- oder Doppelschnecken-Extrusionsvorrichtung ist, optional wobei die Doppelschnecken-Extrusionsvorrichtung ein gleichläufiges oder gegenläufiges System ist.

10. Verfahren nach einem der vorstehenden Ansprüche, ferner umfassend mindestens eines der Folgenden:
i) wobei die Schmelztemperatur des Gemischs zwischen -120 bis +300 °C, typischerweise zwischen 0 und 200 °C, optional zwischen 20 und 180 °C liegt;
ii) wobei das Gemisch ferner ein oder mehrere Funktionsmatrix-Material(ien) umfasst, um das Gemisch zwischen der ersten und der zweiten Substanz zu vermengen und/oder den Schmelzpunkt des Gemischs herunterzusetzen, und/oder dem Endprodukt Funktionen hinzuzufügen, optional wobei das eine oder die mehreren Funktionsmaterial(ien) einen Wirkstoff mit einem Plastifizierungs-Effekt umfassen, wie beispielsweise Polyethylenglycol oder Kohlendioxid oder einen oder mehrere Tenside oder ein oder mehrere Polymere; und
iii) wobei in Schritt b) die Zerstäuberdüse eine Dreh-, Druck-, Flüssigkeits- oder Ultraschall-Konfiguration aufweist.

11. Verfahren nach einem der vorhergehenden Ansprüche, wobei in Schritt c) das Kühlen durch einen Strom von gleichströmendem oder gegenströmendem Kühlgas oder -flüssigkeit, im Verhältnis zur Schmelzsprührichtung, gefördert wird.

12. Verfahren nach einem der vorhergehenden Ansprüche, wobei die Verweildauer der Tröpfchen über ein gegenströmendes Kühlgas oder eine gegenströmende Kühlflüssigkeit gesteuert wird.

13. Verfahren nach einem der Ansprüche 11 oder 12, ferner umfassend eines oder mehrere der folgenden Merkmale:
(i) wobei die Anfangstemperatur des Gas- oder Flüssigkeitsstroms zwischen -20 und 200 °C liegt;
(ii) wobei das gegenströmende Kühlgas Luft, Stickstoff oder Kohlendioxid ist;
(iii) wobei die gegenströmende Kühlflüssigkeit Kohlendioxid oder Flüssigstickstoff ist.

14. Verfahren nach einem der Ansprüche 11, 12 oder 13(i), ferner umfassend eines oder mehrere der folgenden Merkmale:
(i) wobei das Kühlgas Luft, Stickstoff oder Kohlendioxid ist;
(ii) wobei die Kühlflüssigkeit Kohlendioxid oder Flüssigstickstoff ist.

15. Verfahren nach einem der vorhergehenden Ansprüche, ferner umfassend eines oder mehrere der folgenden Merkmale:
(i) wobei die erlangten Partikel mindestens 50 % (Gewichtsprozent) Co-Kristall-Reinheit, noch bevorzugterweise mindestens 75 % (Gewichtsprozent) Co-Kristall-Reinheit, insbesondere 90 % (Gewichtsprozent) oder mehr Co-Kristall-Reinheit umfassen;
(ii) wobei die erhaltenen Teilchen eine Teilchengröße von 1 bis 500 µm aufweisen.

## Revendications

1. Procédé de fabrication de co-cristaux avec au moins une première substance et une deuxième substance qui sont capables de former des co-cristaux, où la première substance est un ingrédient pharmaceutiquement actif (IPA) ou un dérivé pharmaceutiquement acceptable, tel qu'un sel, d'un IPA, lequel procédé comprend les étapes :
a) d'alimentation d'un mélange fondu de l'au moins une première substance et une deuxième substance dans un atomiseur ;
b) d'atomisation du mélange fondu en gouttelettes ;
c) de solidification des gouttelettes en particules ; et
d) de collecte des dites particules.

2. Procédé selon la revendication 1, comprenant en outre une caractéristique suivante :
i) où le procédé est un procédé par lots ou un procédé en continu ;
ii) où les première et deuxième substances sont combinées dans un rapport stoechiométrique, tel que 1 : 1, 1 : 2, 2 : 1 ou un autre rapport de nombres entiers ;
iii) où le procédé est un procédé de fabrication de co-cristaux pharmaceutiques ; ou
iv) dans lequel la première substance, ou son dérivé pharmaceutiquement acceptable, a au moins un groupe fonctionnel choisi parmi : un thioéther, un alcool, un thiol, un aldéhyde, une cétone, une thiocétone, un ester nitrate, un ester phosphate, un ester thiophosphate, un ester, un thioester, un ester sulfate, un acide carboxylique, de l'acide phosphonique, de l'acide phosphinique, de l'acide sulfonique, un amide, une amine primaire, une amine secondaire, de l'ammoniac, une amine tertiaire, une imine, un thiocyanate, un cyanamide, une oxime, un nitrile, un diazo, un halogénure organique, un nitro, un cycle S-hétérocyclique, un thiophène, un cycle N-hétérocyclique, un pyrrole, un cycle O-hétérocyclique, un furane, un époxyde, un peroxyde, de l'acide hydroxamique, un imidazole, et de la pyridine.

3. Procédé selon une quelconque revendication précédente, dans lequel la deuxième substance est un coformeur, éventuellement dans lequel le coformeur est un ingrédient actif ou non actif.

4. Procédé selon la revendication 3, dans lequel le coformeur est un excipient pharmaceutique, une vitamine, un acide minéral ou aminé.

5. Procédé selon l'une quelconque des revendications 3 à 4, dans lequel le coformeur a au moins un groupe fonctionnel choisi parmi : un thioéther, un alcool, un thiol, un aldéhyde, une cétone, une thiocétone, un ester nitrate, un ester phosphate, un ester thiophosphate, un ester, un thioester, un ester sulfate, un acide carboxylique, de l'acide phosphonique, de l'acide phosphinique, de l'acide sulfonique, un amide, une amine primaire, une amine secondaire, de l'ammoniac, une amine tertiaire, une imine, un thiocyanate, un cyanamide, une oxime, un nitrile, un diazo, un halogénure organique, un nitro, un cycle S-hétérocyclique, un thiophène, un cycle N-hétérocyclique, un pyrrole, un cycle O-hétérocyclique, un furane, un époxyde, un peroxyde, de l'acide hydroxamique, un imidazole, et de la pyridine.

6. Procédé selon une quelconque revendication précédente, dans lequel les première et deuxième substances sont exposées à un chauffage pendant au moins 1 minute, éventuellement, 2 minutes ou plus longtemps, jusqu'à ce qu'un mélange homogène et fondu soit formé.

7. Procédé selon une quelconque revendication précédente, dans lequel un cisaillement et/ou une pression sont employés pour faciliter le mélange et l'enchevêtrement des composants dans le mélange, ainsi que pour diminuer leur viscosité, éventuellement, où le cisaillement ou la pression sont assurés par des barreaux d'agitateurs magnétiques, des pales ou par un procédé d'extrusion.

8. Procédé selon la revendication 7, dans lequel le procédé d'extrusion est un appareil d'extrusion basé sur une vis en connexion avec un système d'atomisation.

9. Procédé selon la revendication 8, dans lequel l'appareil d'extrusion basé sur une vis en connexion avec le système d'atomisation est un appareil d'extrusion à vis unique ou à double vis, éventuellement, où l'appareil d'extrusion à double vis est un système en co-rotation ou en contre-rotation.

10. Procédé selon une quelconque revendication précédente, comprenant en outre une ou plusieurs caractéristiques suivantes :
i) où la température de fusion du mélange est entre-120 et +300 °C, typiquement, entre 0 et 200 °C, éventuellement, entre 20 et 180 °C ;
ii) où le mélange comprend en outre un ou plusieurs matériau(x) fonctionnel(s) de matrice pour promouvoir le mélange entre les première et deuxième substances, et/ou pour diminuer la température de fusion du mélange, et/ ou pour ajouter une fonctionnalité au produit final, éventuellement, où le ou les matériau(x) de matrice fonctionnels comprennent un agent avec un effet plastifiant, tel que le polyéthylène glycol, ou du dioxyde de carbone, ou un ou plusieurs tensioactifs, un ou plusieurs polymères ; et
iii) où dans l'étape b), la buse d'atomisation a une configuration en rotation, pour la pression, pour un fluide, ou pour les ultrasons.

11. Procédé selon une quelconque revendication précédente, dans lequel, dans l'étape c), le refroidissement est assuré par un écoulement de gaz ou de liquide de refroidissement dans le sens du courant ou à contre-courant par rapport à la direction du jet de produit fondu.

12. Procédé selon une quelconque revendication précédente, dans lequel le temps de résidence des gouttelettes est commandé par un écoulement de gaz ou de liquide de refroidissement à contre-courant.

13. Procédé selon l'une quelconque des revendications 11 ou 12, comprenant en outre une ou plusieurs des caractéristiques suivantes :
(i) où la température initiale du courant de gaz ou de liquide est entre -20 et 200 °C ;
(ii) où l'écoulement à contre-courant de gaz de refroidissement est de l'air, de l'azote ou du dioxyde de carbone ;
(iii) où l'écoulement à contre-courant de liquide de refroidissement est du dioxyde de carbone liquide ou de l'azote liquide.

14. Procédé selon l'une quelconque des revendications 11, 12 ou 13(i), comprenant en outre une ou plusieurs des caractéristiques suivantes :
(i) où le gaz de refroidissement est de l'air, de l'azote ou du dioxyde de carbone ;
(ii) où le liquide de refroidissement est du dioxyde de carbone liquide ou de l'azote liquide.

15. Procédé selon une quelconque revendication précédente, comprenant en outre une ou plusieurs des caractéristiques suivantes :
(i) où les particules obtenues comprennent au moins 50 % de pureté de co-cristal (p/p), plus préférentiellement au moins 75 % de pureté de co-cristal (p/p), spécialement 90 % ou plus de pureté de co-cristal (p/p) ;
(ii) où les particules obtenues ont une taille particulaire de 1 à 500 µm.
